# EUROPEAN PATENT SPECIFICATION

(11) **EP 3 727 490 B1**
(45) Date of publication and mention of the grant of the patent: **02.07.2025**
(21) Application number: 18826671.2
(22) Date of filing: 20.12.2018
(51) Int. Cl.: A61L 31/04, A61L 31/06, A61L 31/14, D03D 11/00, D03D 27/08, D03D 27/10

(54) **A SELF ADHERING SURGICAL FABRIC AND METHOD OF MANUFACTURING SAME**
SELBSTKLEBENDES CHIRURGISCHES GEWEBE UND VERFAHREN ZU SEINER HERSTELLUNG
TISSU CHIRURGICAL AUTO-ADHÉRENT ET SON PROCÉDÉ DE FABRICATION

(30) Priority: 21.12.2017 IE S20170262
(43) Date of publication of application: 28.10.2020
(73) Proprietor: Aran Biomedical Teoranta, County Galway, H91 C2NF (IE)
(72) Inventor: KING, Dean, Galway (IE); DUFFY, Stephen, Galway (IE)
(74) Representative: FRKelly
(86) International application number: PCT/EP2018/086241
(87) International publication number: WO 2019/122154

(56) References cited:
- EP-A1- 3 628 763
- WO-A1-2010/139340
- WO-A1-93/10731
- GB-A- 1 123 575
- US-A- 1 313 037

## Description

### Field of the invention

This invention relates to a self adhering surgical fabric and a method of manufacturing same, which fabric may be used as a scaffold, support or closure member in various surgical procedures in order to reduce surgical time and complexity.

### Background of the invention

Surgical fabrics or meshes are commonly used in various surgical procedures to provide a permanent or temporary support to organs or other tissue while a particular surgical procedure is performed, for example a hernia repair, pelvic organ prolapse, wound closure, surgical incision closure, vessel puncture site closure, AAA/TAA endovascular grafting to inhibit migration, and integration into oesophageal or biliary covered stents to inhibit migration. The meshes are conventionally manufactured from a polymer and may be inorganic or biological, and are often a bioabsorbable material such that the mesh can be left in situ and will be absorbed into the surrounding tissue over time in order to avoid the requirement for further surgery to remove the mesh.

The mesh is conventionally sutured into position, or retained with a biocompatible adhesive or the like, which procedures add time and complexity to the overall procedure. If the mesh is not fixed in position migration can occur, which can result in secondary complications for example intestinal obstruction in the case of an oesophageal stent.

WO2010139340 discloses a medical device comprising yarns made of a shape memory material and polymer yarns, wherein the yarns made of said shape memory material have a polymer sheathing and having an outer surface including structures to allow anchoring or securing of the device in a surrounding tissue region. WO9310731 discloses a pad to be applied to bleeding tissue and having a textured surface which may include long or short spikes or barbs, or hook like members and/or loop like members.

It is therefore an object of the present invention to address the above-mentioned problem.

### Summary of the invention

According to the present invention there is provided a method of manufacturing a self-adhering surgical fabric comprising the steps of:
a) forming a first layer of material;
b) forming a second layer of material;
c) joining the first and second layers of material in spaced parallel relationship to one another with an array of connecting yarns (16;116) extending to the first and second layers of material; and
d) cutting the connecting yarns (16;116) to separate the first layer of material (12; 112) from the second layer of material (14) such that both of the first and second layers of material (12; 112, 14) retain a respective first and second portion of each connecting yarn (16; 116) projecting from a face of the respective first and second layers of material (12;112, 14) to respective first and second free ends, wherein the free ends formed after cutting the connecting yarns are configured to function as anchors or hooks to self-adhere the respective first and second layers of material to tissue.

Preferably the method comprises weaving or knitting the first and second layers of material (12; 112, 14)

Preferably, the method comprises cutting the connecting yarns using heat and/or mechanical action.

Preferably, the method comprises cutting the connecting yarns using a hot knife. Preferable, the method comprises forming the respective first and second free ends as first and second heads (118) that are configured to function as the anchors or hooks to self-adhere the respective first and second layers of material to tissue Preferably, the method comprises forming the first and second heads (118) simultaneously with cutting the connecting yarn.

Preferably, the method comprises orienting the connecting yarns (16: 116) perpendicularly to the first and second layers of material (12; 112, 14)

Preferably, the method comprises orienting the connecting yarns (16; 116) non-perpendicularly to the first and second layers of material (12; 112, 14).

Preferably, the method comprises interweaving the connecting yarn (16; 116) with the first and second layers of material (12; 112, 14).

Preferably, the method comprises forming the first and second layers of material (12; 112, 14) from one or more polymers.

According to a second aspect of the invention there is provided a self-adhering surgical fabric (10; 110) comprising a layer of a woven material or a knitted material(12; 112, 14) having an array of yarns (16; 116) projecting from a face of the layer of woven or knitted material (12; 112, 14) to an array of mushroom-shaped heads (118) that are configured to function as anchors or hooks to self-adhere the layer of woven or knitted material comprising the surgical fabric to tissue. Preferably, the layer of woven or knitted material (12; 112, 14) is formed from monofilament, multifilament or ring spun yarns of regenerated silk, nylon/high tension nylon, PET/high tension PET, UHMWPE, or LCP (liquid crystal polymer).

Preferably, the first and second layers of material are constructed from resorbable polymers selected from the group of PLA, PLLA, PGA, PLGA, Polycaprolactone or Polydioxanone.

Preferably, each connecting yarn is oriented perpendicularly to the face of the layer of material.

Preferably, each connecting yarn is oriented non perpendicularly to the face of the layer of material.

Preferably, the yarns comprising the first and second layers of material are heat set before being woven or knitted into the first and second layers.

### Brief description of the drawings

The present invention will now be described with reference to the accompanying drawings, in which:
Figure 1 illustrates a knitted dual layered fabric with connecting yarns extending between the two layers;
Figure 2 illustrates the layered fabric of Figure 1 and showing a knife positioned to cut through the middle of the connecting yarns to form two surgical fabrics according to the invention;
Figure 3 illustrates a cross sectional view of Figure 2;
Figure 4 illustrates a self adhering surgical fabric according to the invention as formed from either half of the cut layered fabric of Figures 2 and 3;
Figure 5 illustrates a cross sectional view of Figure 4;
Figure 6a illustrates the surgical fabric of Figures 4 and 5 with a flat hot platen tool being advanced towards the tops of the connecting yarns to form heads thereon;
Figure 6b illustrates the surgical fabric with a hot platen tool with spike shaped depressions being advanced towards the tops of the yarns;
Figure 6c illustrates the surgical fabric with a hot platen tool with dome-shaped depressions being advanced towards the tops of the yarns; and
Figure 7 illustrates a cross sectional view of the surgical fabric with protruding connecting yarns having a dome or mushroom shaped head or barb formed at the free end of each connecting yarn.

### Detailed description of the drawings

Referring now to the accompanying drawings there is illustrated a layered fabric, generally indicated as (10), which comprises a first layer of material (12) and a second layer of material (14) in parallel spaced relationship to one another, and connected together by an array of connecting yarns (16) extending between the layers (12), (14). The connecting yarns (16) may extend perpendicularly from one or both of the layers (12), (14) or may extend at an angle to one or both of the layers (12), (14). The first layer (12) and/or the second layer (14) may be formed as a woven material or as a knitted material, or any other suitable construction. A woven material is produced by orienting yarns along both X and Y axes, that is by arranging a series of warp yarns adjacent one another and interspersing or interweaving weft yarns. The resulting material often tends to offer minimal compliance, depending on the yarn or combination of yarns utilised. It will be appreciated that the physical characteristics of the yarns and the weave may be varied to provided particular desired characteristics such as flexibility, porosity, etc.

The first and/or second layers of material (12), (14) may be constructed from monofilament, multifilament or ring spun yarns of regenerated silk, nylon/high tension nylon, PET/high tension PET, UHMWPE, LCP (liquid crystal polymer) or any other suitable material. Where a multifilament is utilised this may be heat set in a twisted condition or not. The layers of material (12), (14) may also be constructed from resorbable polymers, for example PLA, PLLA, PGA, PLGA, Polycaprolactone or Polydioxanone. In a preferred embodiment illustrated the yarns utilised in the construction of the layers of material (12), (14) can range from 5 to 500 denier.

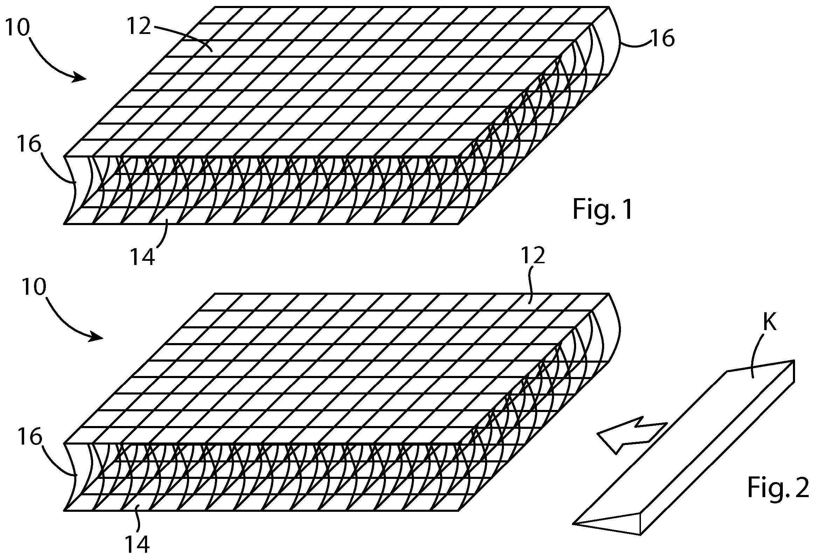

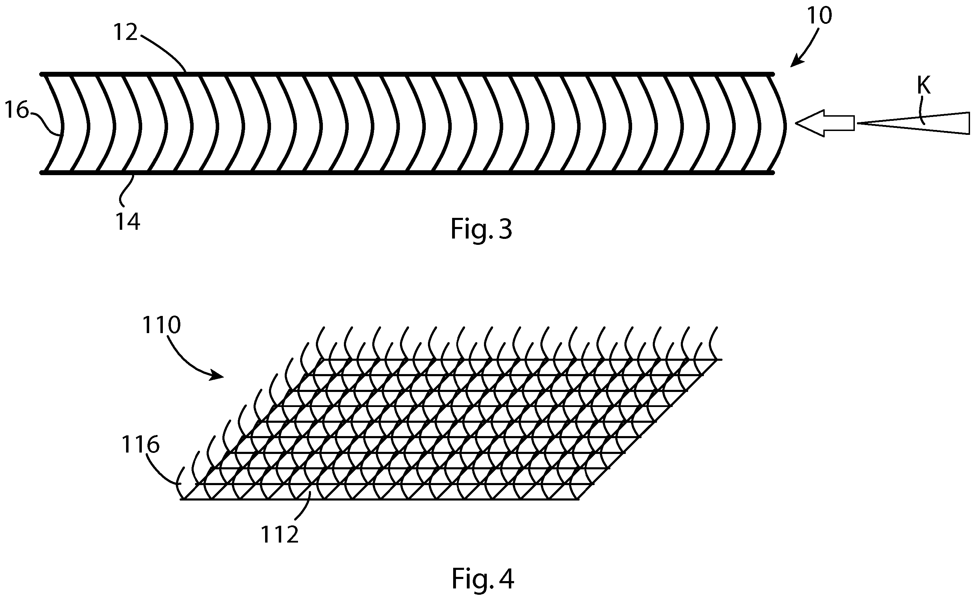

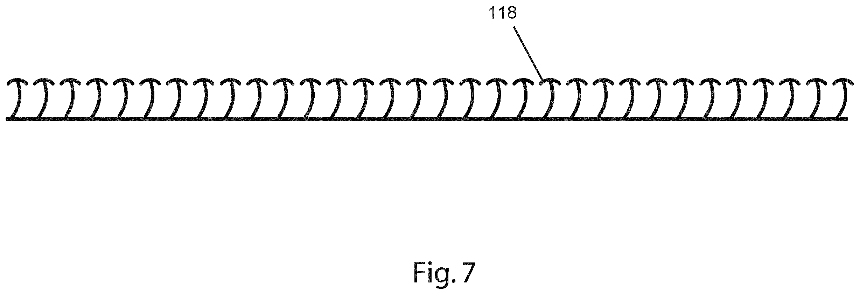

Referring to Figures 2 and 3, once the layered fabric (10) has been constructed, the method according to the present invention then involves separating the first layer of material (12) from the second layer of material (14) by cutting through the connecting yarns (16) such that a portion of each connecting yarn (16) remains attached to both the first layer (12) and to the second layer (14), either of which then define a self adhering surgical fabric (110) according to the invention and as illustrated in Figures 4 to 7. In a preferred embodiment of the method a knife K, which may be heated, is used to cut through the connecting yarns (16) to form the two portions of self adhering fabric (110).

The self adhering surgical fabric (110) comprises a substrate (112) from which projects an array of yarns (116) which function as anchors or hooks which allow the surgical fabric (110) to self adhere to tissue or the like, thereby avoiding the requirement for suturing or otherwise securing the self adhering fabric (110) into position at a surgical site or the like. Traditionally such surgical meshes are required to be sutured or adhered into place, and by avoiding this additional step the self adhering surgical fabric (110) of the present invention reduces the time and complexity of the surgical operation and as a result can improve patient healing response. Potential surgical applications for the self adhering surgical fabric (110) include fixation to treat pelvic organ prolapse, wound closure, surgical incision closure, vessel puncture site closure, AAA/TAA endovascular grafts to inhibit migration, and integration into oesophageal or biliary covered stents to inhibit migration.

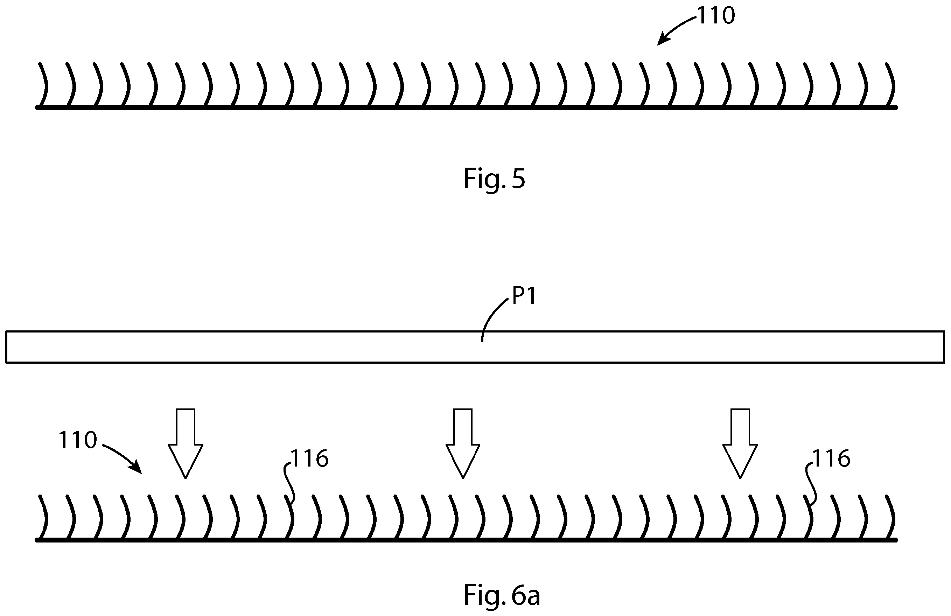

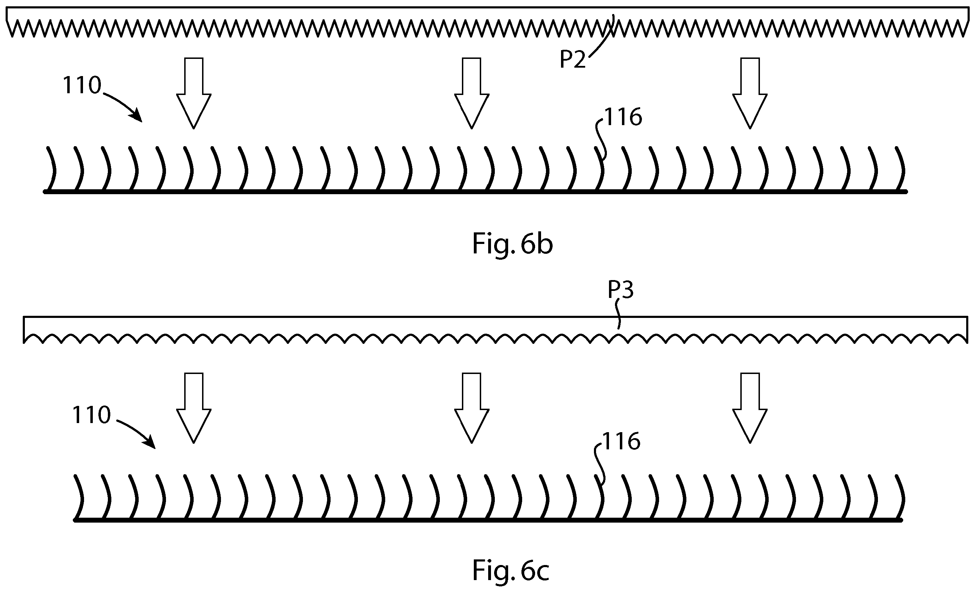

In order to improve the self adherence of the surgical fabric (110) the free end of each of the connecting yarns (116) may be shaped to define a head or barb (118) which will increase the grip generated when the yarns (116) engage with the target tissue. The heads (118) may be formed simultaneously with cutting of the connecting yarns (16) by the knife K, which may be heated in order to partially melt the free end of each connecting yarn (16) as it is cut, thereby deforming the free end of the connecting yarn (16) to form the head (118). Alternatively as illustrated in Figures 6a, 6b and 6c the method of forming the self adhering surgical fabric (110) of the present invention may also include the step of engaging a hot platen P1, P2 or P3 against the free end of each of the yarns (116) in order to melt and deform the free end to form the head (118). The platen P1 comprises a flat contact surface to engage against the yarns (116), while the platens P2 and P3 incorporate a spiked and a domed contact surface in order to generate a barbed or mushroom shaped head (118) when pressed into engagement with the yarns (116). It will of course be appreciated that any other functional alternative may be employed in order to form the shaped heads (118).

It will also be appreciated that the two halves of the layered fabric (10) once separated from one another by the action of the knife K cutting the connecting yarns (116), can be utilised as a self adhering surgical fabric (110) according to the invention.

## Claims

1. A method of manufacturing a self-adhering surgical fabric (10; 110), comprising the steps of:
a) forming a first layer of material (12; 112);
b) forming a second layer of material (14);
c) joining the first and second layers of material (12; 112, 14) in spaced parallel relationship to one another with an array of connecting yarns (16; 116) extending to the first and second layers of material; and
d) cutting the connecting yarns (16; 116) to separate the first layer of material (12; 112) from the second layer of material (14) such that both of the first and second layers of material (12; 112, 14) retain a respective first and second portion of each connecting yarn (16; 116) projecting from a face of the respective first and second layers of material (12; 112, 14) to respective first and second free ends, wherein the free ends formed after cutting the connecting yarns are configured to function as anchors or hooks to self-adhere the respective first and second layers of material to tissue.

2. The method according to claim 1, comprising weaving or knitting the first and second layers of material (12; 112, 14).

3. The method according to any preceding claim, comprising cutting the connecting yarns (16; 116) using heat and/or mechanical action; and optionally cutting the connecting yarns using a hot knife.

4. The method according to any preceding claim, comprising forming the respective first and second free ends as first and second heads (118) that are configured to function as the anchors or hooks to self-adhere the respective first and second layers of material to tissue.

5. The method according to claim 4, comprising forming the first and second heads (118) simultaneously with cutting the connecting yarn.

6. The method according to any preceding claim, comprising orienting the connecting yarns (16; 116) perpendicularly to the first and second layers of material (12; 112, 14).

7. The method according to any preceding claim, comprising orienting the connecting yarns (16; 116) non-perpendicularly to the first and second layers of material (12; 112, 14).

8. The method according to any preceding claim, comprising interweaving the connecting yarn (16; 116) with the first and second layers of material (12; 112, 14).

9. The method according to any preceding claim comprising, forming the first and second layers of material (12; 112, 14) from one or more polymers.

10. A self-adhering surgical fabric (10; 110) comprising a layer of a woven material or a knitted material (12; 112, 14) having an array of yarns (16; 116) projecting from a face of the layer of woven or knitted material (12; 112, 14) to an array of mushroom-shaped heads (118), that are configured to function as anchors or hooks to self-adhere the layer of woven or knitted material comprising the surgical fabric to tissue.

11. The self-adhering surgical fabric according to claim 10, wherein the layer of woven or knitted material (12; 112, 14) is formed from monofilament, multifilament or ring spun yarns of regenerated silk, nylon/high tension nylon, PET/high tension PET, UHMWPE, or LCP (liquid crystal polymer).

12. The self-adhering surgical fabric according to any of claims 10 or 11, wherein the layer of woven or knitted material (12; 112, 14) is constructed from yarns of resorbable polymers selected from the group of PLA, PLLA, PGA, PLGA, Polycaprolactone or Polydioxanone.

13. The self-adhering surgical fabric according to any of claims 10 to 12, wherein the yarns comprising the layer of woven or knitted material (12; 112, 14) have a range of 5 denier to 500 denier.

14. The self-adhering surgical fabric according to any of claims 10 to 13, wherein yarns (16; 116) projecting from the layer of woven or knitted material (12; 112, 14) to the array of mushroom-shaped heads (118) are oriented either perpendicularly or non-perpendicularly to the face of the layer of woven or knitted material (12; 112, 14).

15. The self-adhering surgical fabric according to any of claims 10 to 14, wherein layer of woven or knitted material (12; 112, 14) are a multifilament that is heat set before being woven or knitted into the layer of woven or knitted material (12; 112, 14).

## Patentansprüche

1. Verfahren zum Herstellen eines selbstklebenden chirurgischen Gewebes (10; 110), umfassend die folgenden Schritte:
a) Bilden einer ersten Materialschicht (12; 112);
b) Bilden einer zweiten Materialschicht (14);
c) Verbinden der ersten und zweiten Materialschicht (12; 112, 14) in beabstandeter paralleler Beziehung zueinander mit einer Anordnung von Verbindungsgarnen (16; 116), die sich zu der ersten und zweiten Materialschicht erstrecken; und
d) Schneiden der Verbindungsgarne (16; 116), um die erste Materialschicht (12; 112) derart von der zweiten Materialschicht (14) zu trennen, dass sowohl die erste als auch zweite Materialschicht (12; 112, 14) einen jeweiligen ersten und zweiten Abschnitt jedes Verbindungsgarns (16; 116) zurückhalten, die von einer Fläche der jeweiligen ersten und zweiten Materialschicht (12; 112, 14) zu einem jeweiligen ersten und zweiten freien Ende vorstehen, wobei die freien Enden, die nach dem Schneiden der Verbindungsgarne gebildet werden, konfiguriert sind, um als Anker oder Haken zu fungieren, um die jeweilige erste und zweite Materialschicht selbst an Gewebe zu kleben.

2. Verfahren nach Anspruch 1, umfassend Weben oder Stricken der ersten und zweiten Materialschicht (12; 112, 14).

3. Verfahren nach einem der vorhergehenden Ansprüche, umfassend Schneiden der Verbindungsgarne (16; 116) mithilfe von Wärme und/oder mechanischer Einwirkung; und optional Schneiden der Verbindungsgarne mithilfe eines heißen Messers.

4. Verfahren nach einem der vorhergehenden Ansprüche, umfassend Bilden des jeweiligen ersten und zweiten freien Endes als erster und zweiter Kopf (118), die konfiguriert sind, um als Anker oder Haken zu fungieren, um die jeweilige erste und zweite Materialschicht selbst an Gewebe zu kleben.

5. Verfahren nach Anspruch 4, umfassend gleichzeitiges Bilden des ersten und zweiten Kopfes (118) mit dem Schneiden des Verbindungsgarns.

6. Verfahren nach einem der vorhergehenden Ansprüche, umfassend Ausrichten der Verbindungsgarne (16; 116) senkrecht zu der ersten und zweiten Materialschicht (12; 112, 14).

7. Verfahren nach einem der vorhergehenden Ansprüche, umfassend Ausrichten der Verbindungsgarne (16; 116) nicht senkrecht zu der ersten und zweiten Materialschicht (12; 112, 14).

8. Verfahren nach einem der vorhergehenden Ansprüche, umfassend Verweben des Verbindungsgarns (16; 116) mit der ersten und zweiten Materialschicht (12; 112, 14).

9. Verfahren nach einem der vorhergehenden Ansprüche, umfassend Bilden der ersten und zweiten Materialschicht (12; 112, 14) aus einem oder mehreren Polymeren.

10. Selbstklebendes chirurgisches Gewebe (10; 110), umfassend eine Schicht aus einem gewebten oder gestrickten Material (12; 112, 14), die eine Anordnung von Garnen (16; 116) aufweist, die von einer Fläche der Schicht aus gewebtem oder gestricktem Material (12; 112, 14) zu einer Anordnung von pilzförmigen Köpfen (118) vorstehen, die konfiguriert sind, um als Anker oder Haken zu fungieren, um die Schicht aus gewebtem oder gestricktem Material, umfassend das chirurgische Gewebe, selbst an Gewebe zu kleben.

11. Selbstklebendes chirurgisches Gewebe nach Anspruch 10, wobei die Schicht aus gewebtem oder gestricktem Material (12; 112, 14) aus Monofilament-, Multifilament- oder Ringspinngarnen aus regenerierter Seide, Nylon/Hochspannungsnylon, PET/Hochspannungs-PET, UHMWPE oder LCP (Flüssigkristallpolymer) gebildet ist.

12. Selbstklebendes chirurgisches Gewebe nach einem der Ansprüche 10 oder 11, wobei die Schicht aus gewebtem oder gestricktem Material (12; 112, 14) aus Garnen aus resorbierbaren Polymeren konstruiert ist, die aus der Gruppe aus PLA, PLLA, PGA, PLGA, Polycaprolacton oder Polydioxanon ausgewählt sind.

13. Selbstklebendes chirurgisches Gewebe nach einem der Ansprüche 10 bis 12, wobei die Garne, umfassend die Schicht aus gewebtem oder gewirktem Material (12; 112, 14), einen Bereich von 5 Denier bis 500 Denier aufweisen.

14. Selbstklebender chirurgischer Stoff nach einem der Ansprüche 10 bis 13, wobei Garne (16; 116), die von der Schicht aus gewebtem oder gestricktem Material (12; 112, 14) zu der Anordnung von pilzförmigen Köpfen (118) vorstehen, entweder senkrecht oder nicht senkrecht zu der Fläche der Schicht aus gewebtem oder gestricktem Material (12; 112, 14) ausgerichtet sind.

15. Selbstklebendes chirurgisches Gewebe nach einem der Ansprüche 10 bis 14, wobei die Schicht aus gewebtem oder gestricktem Material (12; 112, 14) ein Multifilament ist, das vor dem Einweben oder Einstricken in die Schicht aus gewebtem oder gestricktem Material (12; 112, 14) thermofixiert wird.

## Revendications

1. Procédé de fabrication d'un tissu chirurgical auto-adhérent (10 ; 110), comprenant les étapes de :
a) formation d'une première couche de matériau (12 ; 112) ;
b) formation d'une deuxième couche de matériau (14) ;
c) jonction des première et deuxième couches de matériau (12 ; 112, 14) dans une relation parallèle espacée l'une par rapport à l'autre avec un réseau de fils de liaison (16 ; 116) s'étendant jusqu'aux première et deuxième couches de matériau ; et
d) coupure des fils de liaison (16 ; 116) pour séparer la première couche de matériau (12 ; 112) de la seconde couche de matériau (14) de telle manière que les première et deuxième couches de matériau (12 ; 112, 14) retiennent une première et une deuxième parties respectives de chaque fil de liaison (16 ; 116) faisant saillie à partir d'une face des première et deuxième couches respectives de matériau (12 ; 112, 14) vers des premières et deuxièmes extrémités libres respectives, les extrémités libres formées après la coupe des fils de liaison étant configurées pour fonctionner comme des ancres ou des crochets pour auto-adhérer les première et deuxième couches de matériau respectives au tissu.

2. Procédé selon la revendication 1, comprenant le tissage ou le tricotage des première et deuxième couches de matériau (12 ; 112, 14).

3. Procédé selon l'une quelconque des revendications précédentes, comprenant la découpe des fils de liaison (16 ; 116) à l'aide de chaleur et/ou d'une action mécanique ; et éventuellement la découpe des fils de liaison à l'aide d'un couteau chaud.

4. Procédé selon l'une quelconque des revendications précédentes, comprenant la formation des premières et deuxièmes extrémités libres respectives en tant que premières et deuxièmes têtes (118) qui sont configurées pour fonctionner comme des ancres ou des crochets pour auto-adhérer les première et deuxième couches de matériau respectives au tissu.

5. Procédé selon la revendication 4, comprenant la formation des première et seconde têtes (118) simultanément à la coupe du fil de liaison.

6. Procédé selon l'une quelconque des revendications précédentes, comprenant l'orientation des fils de liaison (16 ; 116) perpendiculairement aux première et deuxième couches de matériau (12 ; 112, 14).

7. Procédé selon l'une quelconque des revendications précédentes, comprenant l'orientation des fils de liaison (16 ; 116) non perpendiculairement aux première et deuxième couches de matériau (12 ; 112, 14).

8. Procédé selon l'une quelconque des revendications précédentes, comprenant l'entrelacement du fil de liaison (16 ; 116) avec les première et deuxième couches de matériau (12 ; 112, 14).

9. Procédé selon l'une quelconque des revendications précédentes, comprenant la formation des première et deuxième couches de matériau (12 ; 112, 14) à partir d'un ou plusieurs polymères.

10. Tissu chirurgical auto-adhérent (10 ; 110) comprenant une couche d'un matériau tissé ou d'un matériau tricoté (12 ; 112, 14) presentant un réseau de fils (16 ; 116) faisant saillie à partir d'une face de la couche de matériau tissé ou tricoté (12 ; 112, 14) vers un réseau de têtes en forme de champignon (118), qui sont configurées pour fonctionner comme des ancres ou des crochets pour auto-adhérer la couche de matériau tissé ou tricoté comprenant le tissu chirurgical au tissu.

11. Tissu chirurgical auto-adhérent selon la revendication 10, dans lequel la couche de matériau tissé ou tricoté (12 ; 112, 14) est formée à partir de fils monofilaments, multifilaments ou filés à anneaux de soie régénérée, de nylon/nylon haute tension, de PET/PET haute tension, d'UHMWPE ou de LCP (polymère à cristaux liquides).

12. Tissu chirurgical auto-adhérent selon l'une quelconque des revendications 10 ou 11, dans lequel la couche de matériau tissé ou tricoté (12 ; 112, 14) est construite à partir de fils de polymères résorbables choisis dans le groupe de PLA, PLLA, PGA, PLGA, Polycaprolactone ou Polydioxanone.

13. Tissu chirurgical auto-adhésif selon l'une quelconque des revendications 10 à 12, dans lequel les fils constituant la couche de matériau tissé ou tricoté (12 ; 112, 14) ont une plage de 5 deniers à 500 deniers.

14. Tissu chirurgical auto-adhérent selon l'une quelconque des revendications 10 à 13, dans lequel des fils (16 ; 116) faisant saillie de la couche de matériau tissé ou tricoté (12 ; 112, 14) vers le réseau de têtes en forme de champignon (118) sont orientés perpendiculairement ou non perpendiculairement à la face de la couche de matériau tissé ou tricoté (12 ; 112, 14).

15. Tissu chirurgical auto-adhérent selon l'une quelconque des revendications 10 à 14, dans lequel la couche de matériau tissé ou tricoté (12 ; 112, 14) est un multifilament qui est thermofixé avant d'être tissé ou tricoté dans la couche de matériau tissé ou tricoté (12 ; 112, 14).
